(19) Europäisches Patentamt
European Patent Office
Office européen des brevets

(11) Numéro de publication: **0 042 322**
**B1**

(12) **FASCICULE DE BREVET EUROPEEN**

(45) Date de publication du fascicule du brevet: **18.04.84**

(51) Int. Cl.³: **C 07 D 211/12**, C 07 D 211/18, C 07 D 211/22, C 07 D 211/24, A 61 K 31/445

(21) Numéro de dépôt: **81400897.5**

(22) Date de dépôt: **05.06.81**

# (54) Nouveaux dérivés de l'indène, procédés pour leur préparation et des médicaments les contenant.

(30) Priorité: **13.06.80 FR 8013145**

(43) Date de publication de la demande: **23.12.81 Bulletin 81/51**

(45) Mention de la délivrance du brevet: **18.04.84 Bulletin 84/16**

(84) Etats contractants désignés: **BE CH DE FR GB IT LI LU NL SE**

(56) Documents cités:
**BE - A - 881 624**
**FR - A - 2 269 928**
**GB - A - 110 087**
**US - A - 3 340 268**
**US - A - 3 984 407**

(73) Titulaire: **PHARMUKA LABORATOIRES, 35 Quai du Moulin de Cage, F-92231 Gennevilliers (FR)**

(72) Inventeur: **Dubroeucq, Marie-Christine, 13, Villa Malleville, F-95880 Enghien-les-Bains (FR)**
Inventeur: **Gueremy, Claude Georges Alexandre, 3, rue Daumesnil, F-78800 Houilles (FR)**
Inventeur: **Renault, Christian Louis Albert, 10 Allée Réaumur, F-95150 Taverny (FR)**
Inventeur: **Le Fur, Gérard Roger, 35, rue du Progrès, F-92350 Plessis Robinson (FR)**

(74) Mandataire: **Gaumont, Robert et al, RHONE-POULENC RECHERCHES Service Brevets Pharma 25, Quai Paul Doumer, F-92408 Courbevoie Cedex (FR)**

EP 0 042 322 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

# Nouveaux dérivés de l'indène, procédés pour leur préparation et des médicaments les contenant

La présente invention a pour objet de nouveaux dérivés de l'indène utilisables comme médicaments, notamment comme méeicaments pour le traitement des états de dépression, comme analgésiques et antimigraineux. Le brevet US-A No 3984407 divulgue certains dérivés d'indantétrol, leur activité comme antihypertoniques et leurs produits intermédiaires. Le brevet BE-A No 881624 enseigne la préparation de certains dérivés de 4-(naphtylméthyle)pipéridine ayant une activité antiagressive et antischizophrénique.

Ces composés peuvent être représentés par la formule générale:

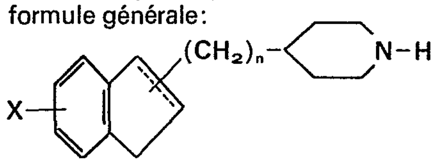
(I)

dans laquelle n est un nombre entier égal à 1, 2 ou 3, X est fixé en position 4, 5, 6 ou 7 sur le cycle

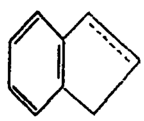

et représente un atome d'hydrogène, un atome d'halogène (en particulier de chlore ou de fluor) ou un groupe alkyle, alcoxy, ou alkylthio ayant 1 à 4 atomes de carbone, le groupe

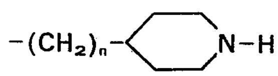

est fixé en position 2 ou 3 sur le cycle

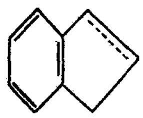

et le trait pointillé représente une deuxième liaison éventuelle.

Comme l'indique la formule (I), les composés selon l'invention dérivent soit de l'indène, auquel cas ils répondent aux formules:

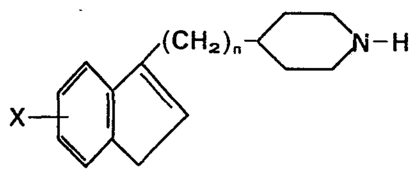
(II)

ou

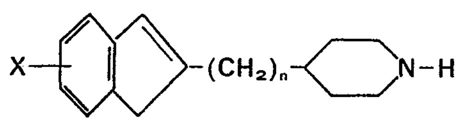
(III)

soit du dihydro-2,3 1H-indène ou indane, auquel cas ils répondent à la formule:

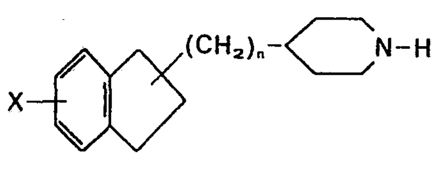
(IV)

Dans les formules (I), (II), (III) et (IV), X est de préférence un atome d'hydrogène.

Les composés de formule (II) peuvent être préparés par réaction d'un dérivé de l'indène de formule:

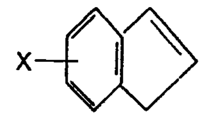

dans laquelle X a la même signification que dans la formule (I), avec un dérivé métallique de formule RM, dans laquelle M désigne un métal alcalin (en particulier le lithium, le sodium, le potassium) et R désigne un atome d'hydrogène, un groupe $NH_2$, un groupe amino mono- ou disubstitué, un groupe alkyle ou un groupe aryle, réaction du composé de formule (V) ainsi obtenu avec un dérivé pipéridinique de formule:

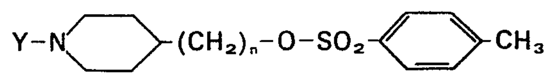
(VI)

dans laquelle n est 1, 2 ou 3 et Y représente un groupe protecteur clivable en milieu acide (en particulier le groupe triphénylméthyle), et action d'un acide sur le composé de formule (VII) ainsi obtenu. L'ensemble des réactions peut être schématisé comme suit:

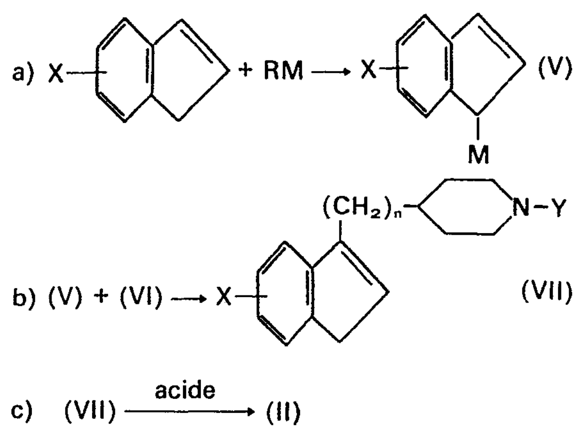

a) + RM (V)
b) (V) + (VI) (VII)
c) (VII) (II)

La réaction a est effectuée au sein d'un solvant inerte, de préférence un éther tel que l'oxyde de diéthyle, le tétrahydrofuranne ou le diméthoxyéthane, à une température comprise entre 0°C et la température d'ébullition du solvant utilisé. Comme dérivé métallique RM, on utilise de préférence un dérivé du lithium, par exemple le phényllithium, le butyllithium ou le diisopropylamidure de lithium, auquel cas la réaction a est effectuée à basse température (de 0°C à la température ambiante).

La réaction de condensation b est effectuée dans les mêmes solvants que la réaction a et à une température comprise entre 0°C et la température d'ébullition du solvant. Lorsqu'on utilise comme dérivé métallique RM un dérivé du lithium, la réaction b est effectuée à une température comprise entre 0°C et la température ambiante.

La réaction c, qui consiste à remplacer le groupe protecteur clivable en milieu acide Y par un atome d'hydrogène, est effectuée selon des méthodes connues en soi. Par exemple, lorsque Y est le groupe triphénylméthyle, la réaction c peut être effectuée en traitant le composé (VII) par une solution hydroalcoolique d'un acide tel que l'acide chlorhydrique, à une température de 20 à 70°C.

Les dérivés pipéridiniques de formule (VI) pour lesquels Y est le groupe triphénylméthyle peuvent être préparés par action du chlorure de triphénylméthyle sur les esters de formule:

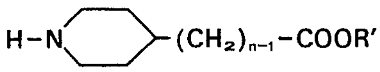 (VIII)

dans laquelle n est égal à 1, 2 ou 3 et R' représente un groupe alkyle de bas poids moléculaire, par exemple méthyle ou éthyle, réduction par l'hydrure de lithium et d'aluminium $LiAlH_4$ des esters de formule:

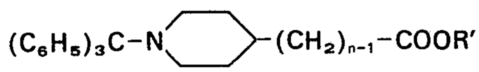 (IX)

ainsi obtenus, et action du chlorure de tosyle sur les alcools de formule:

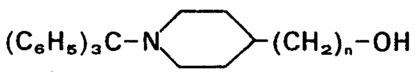 (X)

obtenus.

La réaction du chlorure de triphénylméthyle avec les esters de formule (VIII) est effectuée avantageusement à une température de 20 à 25°C, en présence d'une base (de préférence une amine tertiaire telle que la triéthylamine) et d'un solvant inerte permettant la solubilisation des réactifs, par exemple le chloroforme.

La réduction des esters de formule (IX) par $LiAlH_4$ est effectuée au sein d'un solvant inerte, de préférence un éther tel que l'oxyde de diéthyle ou le tétrahydrofuranne, à une température comprise entre 0°C et la température ambiante.

La réaction du chlorure de tosyle avec les alcools de formule (X) est effectuée à une température comprise entre 0°C et la température ambiante, au sein d'un solvant inerte et en présence d'une base (de préférence une amine). Une méthode avantageuse consiste à opérer au sein de la pyridine qui joue à la fois le rôle de solvant et de base.

Les composés de formule (III) peuvent être préparés par déshydratation en milieu acide des aminoalcools de formule (XI) ci-dessous, dans laquelle X et n ont la même signification que dans la formule (I), Z représente un atome d'hydrogène ou un groupe clivable en milieu acide (par exemple le groupe triphénylméthyle) et le groupe OH est indifféremment en position cis ou en position trans par rapport au groupe

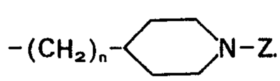

La réaction peut être schématisée comme suit:

d) 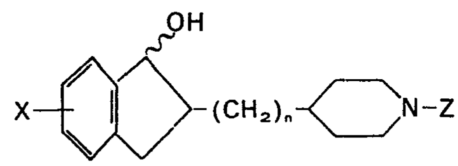

(XI) déshydratation (III)

La réaction d est effectuée selon des méthodes, connues en soi, qui permettent de déshydrater en milieu acide un alcool et de le transformer ainsi en oléfine, par exemple celles décrites par R.B. Wagner et H.D. Zook, «Synthetic Organic Chemistry», p. 32, J. Wiley and Sons, 1965. Une méthode avantageuse consiste à traiter les aminoalcools de formule (XI) par une solution aqueuse 2N à 6N d'acide sulfurique ou par une solution hydroalcoolique d'acide chlorhydrique, à une température de 40 à 80°C.

Les aminoalcools de formule (XI) peuvent être préparés par réduction des cétones de formule:

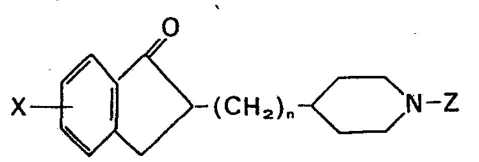

dans laquelle X et n ont la même signification que dans la formule (I) et Z a la même signification que dans la formule (XI). Pour effectuer cette réduction, on utilise comme agent réducteur un hydrure tel que l'hydrure d'aluminium et de lithium au sein d'un solvant inerte, de préférence un éther tel que l'oxyde de diéthyle ou le tétrahydrofuranne. La réaction est effectuée à une température comprise entre 0°C et la température d'ébullition du solvant.

Les composés de formule (IV) peuvent être préparés par hydrogénation catalytique des composés de formules (II) et (III) ou de leurs sels. Cette hydrogénation est effectuée au sein d'un solvant inerte, à une température comprise entre 20 et 80°C et sous une pression d'hydrogène de 1 à 50 bar. Comme solvant, on peut utiliser par exemple des alcools, tels que le méthanol ou l'éthanol, ou des acides tels que l'acide acétique. Comme catalyseur d'hydrogénation, on peut utiliser le nickel, le palladium, le rhodium, le ruthénium ou le platine. Lorsque X représente un atome de chlore, il est avantageux d'utiliser comme solvant l'acide acétique, comme catalyseur le platine et d'opérer sous une pression d'hydrogène égale à la pression atmosphérique.

Les composés de formule (IV) peuvent également être préparés par hydrogénation catalytique, dans des conditions identiques à celles employées pour l'hydrogénation des composés de formules (II) et (III), des composés de formule:

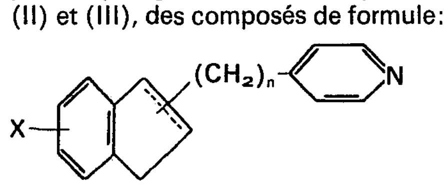 (XII)

dans laquelle X et n ont la même signification que dans la formule (I), le groupe

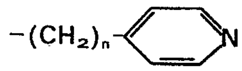

est fixé en position 2 ou 3 sur le cycle

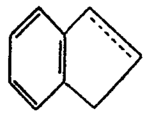

et le trait pointillé représente une deuxième liaison éventuelle.

Les mélanges réactionnels obtenus par les divers procédés décrits précédemment sont traités suivant des méthodes classiques, physiques (évaporation, extraction à l'aide d'un solvant, distillation, cristallisation, chromatographie, etc.) ou chimiques (formation de sel et régénération de la base, etc.) afin d'isoler les composés de formule (I) à l'état pur.

Les composés de formule (I) sous forme de base libre peuvent éventuellement être transformés en sels d'addition avec un acide minéral ou organique par action d'un tel acide au sein d'un solvant approprié.

Les exemples suivants illustrent l'invention sans la limiter. Les données relatives aux spectres de résonance magnétique nucléaire (en abrégé RMN) figurant dans ces exemples concernent la résonance magnétique nucléaire des protons des composés à l'état de base en solution dans le deutérochloroforme (sauf mention contraire). Les déplacements chimiques δ sont mesurés en utilisant comme référence le tétraméthylsilane.

*Exemple I: [(1H-Indényl-3) méthyl]-4 pipéridine*

*1. Préparation du (triphénylméthyl-1 pipéridine-4) carboxylate d'éthyle*

On agite 19 h à la température ambiante une solution de 50 g de pipéridine-4 carboxylate d'éthyle, 93,5 g de chlorure de triphénylméthyle et 97 ml de triéthylamine dans 500 ml de chloroforme. On évapore le solvant sous pression réduite, reprend le résidu dans 600 ml d'éther, filtre l'insoluble et évapore le filtrat. On obtient un résidu huileux que l'on reprend par 600 ml d'éther de pétrole. On filtre le précipité obtenu et obtient ainsi 80 g de (triphénylméthyl-1 pipéridine-4) carboxylate d'éthyle, qui fond à 151°C.

Spectre RMN du produit obtenu:

| | |
|---|---|
| protons des noyaux aromatiques | δ: 7 à 7,5 ppm |
| $-O-\underline{CH_2}-CH_3$ | δ: 4,1 ppm |
| $-O-CH_2-\underline{CH_3}$ | δ: 1,2 ppm |

*2. Préparation du (triphénylméthyl-1 pipéridine-4) méthanol*

A une suspension de 1,9 g d'hydrure de lithium et d'aluminium dans 40 ml de tétrahydrofuranne anhydre, placée sous atmosphère d'azote, on ajoute une solution de 10 g de (triphénylméthyl-1 pipéridine-4) carboxylate d'éthyle dans 120 ml de tétrahydrofuranne anhydre. Après 3 h d'agitation à la température ambiante, on hydrolyse le mélange réactionnel par addition de 10 ml d'eau et 1 ml d'une solution aqueuse 5N d'hydroxyde de sodium. On filtre et évapore le filtrat sous pression réduite. On obtient un résidu huileux que l'on reprend par de l'éther de pétrole. On filtre le précipité obtenu et obtient ainsi 9,8 g de (triphénylméthyl-1 pipéridine-4) méthanol, qui fond à 139°C.

Spectre RMN du produit obtenu:

| | |
|---|---|
| protons des noyaux aromatiques | δ: 7 à 7,5 ppm |
| $-\underline{CH_2}-OH$ | δ: 3,4 ppm |

*3. Préparation de la p-tolylsulfonyloxyméthyl-4 triphénylméthyl-1 pipéridine*

A 9,4 g de (triphénylméthyl-1 pipéridine-4) méthanol dans 95 ml de pyridine on ajoute, à 0°C, 9,6 g de chlorure de p-méthylphénylsulfonyle. Après 3 h d'agitation à la température ambiante, on verse le mélange réactionnel dans 1 l d'eau contenant 75 ml d'éthanol absolu. On agite 2 h, filtre le précipité, le lave à l'eau et le redissout dans du chloroforme. On lave la phase organique à l'eau, la sèche sur du sulfate de sodium et l'évapore sous pression réduite. Le résidu huileux obtenu est repris par de l'éther de pétrole. On filtre le précipité obtenu et obtient ainsi 10 g de p-tolylsulfonyloxyméthyl-4 triphénylméthyl-1 pipéridine, qui fond à 192°C.

Spectre RMN du produit obtenu:

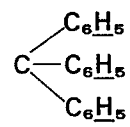 + 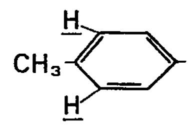 δ: 7 à 7,5 ppm

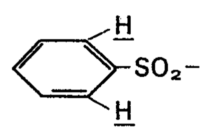 δ: 7,7 ppm

$-\underline{CH_2}-O-$ δ: 3,9 ppm

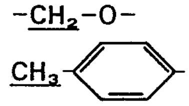 δ: 2,4 ppm

*4. Préparation de la [(1H-indényl-3) méthyl]-4 pipéridine*

A une solution de 10,5 g d'indène dans 100 ml de tétrahydrofuranne, refroidie à 0°C et placée sous atmosphère d'azote, on ajoute goutte à goutte en 30 min 48 ml d'une solution 2M de butyllithium dans l'hexane. L'addition terminée, le mélange réactionnel est agité pendant 2 h à la température ambiante, puis refroidi à 0°C. On ajoute alors en 1 h une solution de 30,8 g de triphénylméthyl-1 p-tolylsulfonyloxyméthyl-4 pipéridine dans 180 ml de tétrahydrofuranne. L'addition terminée, on ajoute 50 ml d'eau, évapore le tétrahydrofuranne sous pression réduite et extrait le résidu par 300 ml d'éther. La phase éthérée est lavée à l'eau, séchée sur sulfate de magnésium et concentrée.

Le résidu obtenu (30 g) est dissous dans un mélange de 300 ml d'éthanol et de 150 ml d'une solution aqueuse 5N d'acide chlorhydrique. La solution obtenue est chauffée pendant 1½ h à 45°C, puis l'éthanol est évaporé sous pression réduite. La phase aqueuse résiduelle est extraite

par 3 fois 200 ml d'éther, puis amenée à pH 9 par addition d'hydroxyde de sodium. L'huile qui relargue est extraite par de l'acétate d'éthyle. Les extraits à l'acétate d'éthyle sont séchés sur sulfate de magnésium et évaporés.

Le résidu huileux obtenu (9,5 g) est fixé sur une colonne contenant 300 g de silice, puis on élue avec un mélange chloroforme/diéthylamine ⁹⁰⁄₁₀. On obtient ainsi 5,6 g de [(1H-indényl-3) méthyl]-4 pipéridine qui, après traitement par une solution d'acide chlorhydrique dans l'acétone, fournissent 6 g de chlorhydrate de [(1H-indényl-3) méthyl]-4 pipéridine. Ce dernier produit fond à 233°C.

*Exemple II: [(1H-Indényl-3)-2 éthyl]-4 pipéridine*

*1. Préparation du (triphénylméthyl-1 pipéridine-4) acétate de méthyle*

On opère comme dans la première partie de l'exemple I, en partant de 80 g de chlorhydrate de pipéridine-4 acétate de méthyle, 142 g de chlorure de triphénylméthyle et 200 ml de triéthylamine dans 1 l de chloroforme. On obtient 131 g de (triphénylméthyl-1 pipéridine-4) acétate de méthyle, qui fond à 164°C.

Spectre RMN du produit obtenu:

protons des noyaux aromatiques δ: 7 à 7,5 ppm

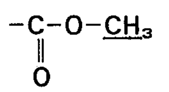 δ: 3,6 ppm

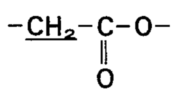 δ: 2,2 ppm

*2. Préparation du (triphénylméthyl-1 pipéridine-4) éthanol*

On opère comme dans la deuxième partie de l'exemple I, en partant de 3,5 g d'hydrure de lithium et d'aluminium et 18,4 g de (triphénylméthyl-1 pipéridine-4) acétate de méthyle dans 300 ml de tétrahydrofuranne anhydre. On obtient 12,5 g de (triphénylméthyl-1 pipéridine-4) éthanol, qui fond à 137°C.

Spectre RMN du produit obtenu:

protons des noyaux aromatiques δ: 7 à 7,5 ppm
$-\underline{CH_2}-OH$ δ: 3,5 ppm

*3. Préparation de la (p-tolylsulfonyloxy-2 éthyl)-4 triphénylméthyl-1 pipéridine*

On opère comme dans la troisième partie de l'exemple I, en partant de 12 g de (triphénylméthyl-1 pipéridine-4) éthanol et 12,5 g de chlorure de p-méthylphénylsulfonyle dans 125 ml de pyridine. On obtient 17,5 g de (p-tolylsulfonyloxy-2 éthyl)-4 triphénylméthyl-1 pipéridine, qui fond à 176°C.

Spectre RMN du produit obtenu:

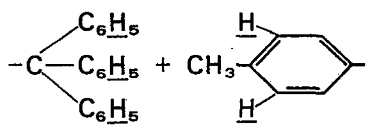 δ: 7 à 7,5 ppm

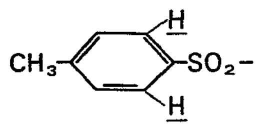 δ: 7,7 ppm

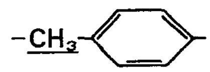 δ: 2,4 ppm

$-\underline{CH_2}-O-SO_2-$ δ: 3,9 ppm

*4. Préparation de la [(1H-indényl-3)-2 éthyl]-4 pipéridine*

On opère comme dans la quatrième partie de l'exemple I, en employant 9,85 g d'indène, 45 ml de solution 2M de butyllithium dans l'hexane et, à la place des 30,8 g de triphénylméthyl-1 p-tolylsulfonyloxyméthyl-4 pipéridine, 29,8 g de triphénylméthyl-1 (p-tolylsulfonyloxy-2 éthyl)-4 pipéridine. On obtient 3 g de chlorhydrate de [(1H-indényl-3)-2 éthyl]-4 pipéridine, qui fond à 199°C.

*Exemple III: [(1H-Indényl-3)-3 propyl]-4 pipéridine*

*1. Préparation du (triphénylméthyl-1 pipéridine-4)-3 propionate d'éthyle*

On opère comme dans la première partie de l'exemple I, en partant de 57,7 g de (pipéridine-4)-3 propionate d'éthyle, 86,9 g de chlorure de triphénylméthyle et 54 ml de triéthylamine dans 600 ml de chloroforme. On obtient 125 g de (triphénylméthyl-1 pipéridine-4)-3 propionate d'éthyle, sous la forme d'une huile.

Spectre RMN du produit obtenu:

protons des noyaux aromatiques δ: 7 à 7,5 ppm
$-\underline{CH_2}-COOC_2H_5$ δ: 2,3 ppm
$-O-\underline{CH_2}-CH_3$ δ: 4,1 ppm

*2. Préparation du (triphénylméthyl-1 pipéridine-4)-3 propanol*

On opère comme dans la deuxième partie de l'exemple I, en partant de 1,9 g d'hydrure de lithium et d'aluminium et 12,6 g de (triphénylméthyl-1 pipéridine-4)-3 propionate d'éthyle dans 150 ml de tétrahydrofuranne anhydre. On obtient 6,2 g de (triphénylméthyl-1 pipéridine-4)-3 propanol, qui fond à 124°C.

Spectre RMN du produit obtenu:

aromatiques δ: 7 à 7,5 ppm
$-\underline{CH_2}-OH$ δ: 3,5 ppm

*3. Préparation de la (p-tolylsulfonyloxy-3 propyl)-4 triphénylméthyl-1 pipéridine*

On opère comme dans la troisième partie de l'exemple I, en partant de 5,95 g de (triphénylméthyl-1 pipéridine-4)-3 propanol et 5,9 g de chlorure de p-méthylphénylsulfonyle dans 60 ml de pyridine. On obtient 7,6 g de (p-tolylsulfonyloxy-3 propyl)-4 triphénylméthyl-1 pipéridine, qui fond à 131°C.

Spectre RMN du produit obtenu:

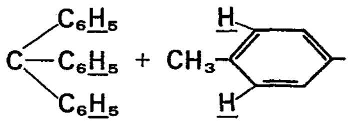 δ: 7 à 7,5 ppm

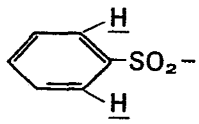 δ: 7,7 ppm

$-CH_2-O-SO_2-$ δ: 3,9 ppm

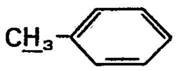 δ: 2,4 ppm

*4. Préparation de la [(1H-indényl-3)-3 propyl]-4 pipéridine*

On opère comme dans la quatrième partie de l'exemple I, en employant 11,3 g d'indène, 52 ml de solution 2M de butyllithium dans l'hexane et, à la place des 30,8 g de triphénylméthyl-1 p-tolylsulfonyloxyméthyl-4 pipéridine, 35,1 g de triphénylméthyl-1 (p-tolylsulfonyloxy-3 propyl)-4 pipéridine. On obtient 9 g de chlorhydrate de [(1H-indényl-3)-3 propyl]-4 pipéridine, qui fond à 170°C.

*Exemple IV: [(1H-Indényl-2) méthyl]-4 pipéridine*

*1. Préparation de la (pipéridinyl-4 méthyl)-2 dihydro-2,3 1H-Indénone-1 (ou (pipéridinyl-4 méthyl)-2 indanone-1).*

60 g de (pyridyl-4 méthyl)-2 dihydro-2,3 1H-indénone-1 en solution dans 600 ml d'acide acétique sont hydrogénés à température ambiante, sous une pression d'hydrogène de 1 bar, en présence de 3 g d'oxyde de platine Adams. Après 7 h d'hydrogénation, le catalyseur est éliminé par filtration et le filtrat est évaporé. Le résidu est fixé sur une colonne contenant 1600 g de silice et on élue avec un mélange chloroforme/diéthylamine 90/10. On obtient ainsi 30,2 g d'un produit purifié, à l'état de base libre. Ce produit est transformé en chlorhydrate. Après deux recristallisations dudit chlorhydrate dans l'éthanol, on obtient 13,3 g de chlorhydrate de (pipéridinyl-4 méthyl)-2 dihydro-2,3 1H-indénone-1, qui fond à 223°C.

*2. Préparation du (pipéridinyl-4 méthyl)-2 dihydro-2,3 1H-indénol-1 (ou (pipéridinyl-4 méthyl)-2 indanol-1)*

A une suspension de 0,38 g d'hydrure d'aluminium et de lithium dans 24 ml de tétrahydrofuranne, refroidie à 0°C et placée sous atmosphère d'azote, on ajoute en 15 min une solution de 2,4 g de (pipéridinyl-4 méthyl)-2 dihydro-2,3 1H-indénone-1 dans 24 ml de tétrahydrofuranne. On agite 1 h à 0°C, puis on ajoute successivement 0,45 ml d'eau, 0,28 ml d'une solution aqueuse 5N d'hydroxyde de sodium et 1,25 ml d'eau. Les produits minéraux sont filtrés, lavés par du chlorure de méthylène. Le filtrat et les lavages sont rassemblés et évaporés. On obtient ainsi 2,3 g de (pipéridinyl-4 méthyl)-2 dihydro-2,3 1H-indénol-1 sous la forme d'un mélange à 75% d'isomère cis et 25% d'isomère trans.

Spectre RMN du produit obtenu:

Le produit obtenu étant représenté par la formule globale:

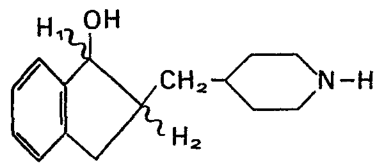

les caractéristiques spectrales des protons $H_1$ et $H_2$ fixés sur le cycle sont les suivantes:

pour l'isomère trans: $H_1$ δ = 4,9 ppm
$J_{H_1H_2}$ = 5 Hz
pour l'isomère cis: $H_1$ δ = 4,7 ppm
$J_{H_1H_2}$ = 7 Hz

*3. Préparation de la [(1H-indényl-2) méthyl]-4 pipéridine*

Une solution de 2,3 g de (pipéridinyl-4 méthyl)-2 dihydro-2,3 1H-indénol-1 dans 25 ml d'une solution aqueuse 4N d'acide sulfurique est chauffée à 60°C pendant 2 h. La solution, refroidie à température ambiante, est amenée à pH 10 par addition d'hydroxyde de sodium. L'huile qui relargue est extraite à l'acétate d'éthyle. L'extrait organique est lavé à l'eau, séché sur sulfate de magnésium et évaporé. Le résidu huileux est fixé sur une colonne contenant 60 g de silice et on élue avec un mélange de 90 parties en volume de chloroforme et 10 parties en volume de diéthylamine (mélange chloroforme/diéthylamine 90/10). On obtient ainsi 1,25 g de [(1H-indényl-2) méthyl]-4 pipéridine à l'état cristallisé, que l'on dissout dans 12 ml d'acétone et traite par 0,85 ml d'une solution 7N d'acide chlorhydrique dans l'éther. On obtient alors 1,35 g de chlorhydrate de [(1H-indényl-2) méthyl]-4 pipéridine, qui fond à 234°C.

*Exemple V: [(1H-Indényl-2)-3 propyl]-4 pipéridine*

*1. Préparation de la [(triphénylméthyl-1 pipéridinyl-4)-3 propyl]-2 dihydro-2,3 1H-indénone-1*

A une solution de 29 g de dihydro-2,3 1H-indénone-1 dans 240 ml de diméthoxyéthane, placée sous atmosphère d'azote, on ajoute par portions 21 g d'hydrure de sodium (sous la forme d'une suspension à 50% dans l'huile de paraffine) puis, goutte à goutte, en 45 min, une solution de 27 g de triphénylméthyl-1 (iodo-3 propyl)-4 pipéridine dans 240 ml de diméthoxyéthane. Après l'addition, on agite à température ambiante pendant 30 min, refroidit au bain de glace et ajoute avec précaution 100 ml d'eau, puis 22 ml d'acide acétique, puis 800 ml d'eau. On extrait à l'éther, sèche l'extrait éthéré sur sulfate de magnésium et évapore l'éther. Le résidu obtenu (58 g) est fixé sur une colonne contenant 2000 g de silice et on élue avec un mélange de 90 parties en volume de cyclohexane et 10 parties en volume d'acétate d'éthyle.

On obtient ainsi 16,5 g de [(triphénylméthyl-1 pipéridinyl-4)-3 propyl]-2 dihydro-2,3 1H-indénone-1.

Spectre RMN du produit obtenu:

Le produit obtenu étant représenté par la formule:

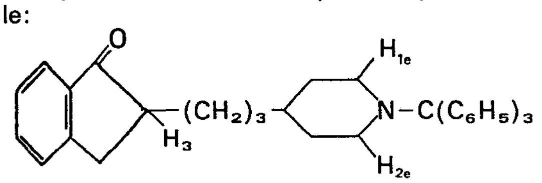

les caractéristiques spectrales des protons numérotés $H_{1e}$, $H_{2e}$, $H_3$ sont les suivantes:

$\underline{H}_{1e}$, $\underline{H}_{2e}$, $\underline{H}_3$ δ: 3 ppm (multiplet)

*2. Préparation du [(triphénylméthyl-1 pipéridinyl-4)-3 propyl]-2 dihydro-2,3 1H-indénol-1*

On opère comme dans la deuxième partie de l'exemple IV, en partant de 1,14 g d'hydrure d'aluminium et de lithium dans 150 ml de tétrahydrofuranne et 15 g de [(triphénylméthyl-1 pipéridinyl-4)-3 propyl]-2 dihydro-2,3 1H-indénone-1 dans 150 ml de tétrahydrofuranne. On obtient ainsi 14,8 g de [(triphénylméthyl-1 pipéridinyl-4)-3 propyl]-2 dihydro-2,3 1H-indénol-1 sous la forme d'un mélange à 70% d'isomère cis et 30% d'isomère trans.

Spectre RMN du produit obtenu:

Le produit obtenu étant représenté par la formule globale:

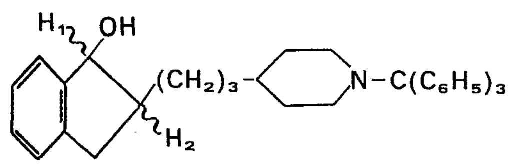

les caractéristiques spectrales des protons $H_1$ et $H_2$ fixés sur le cycle sont les suivantes:

pour l'isomère trans: $\underline{H}_1$ δ = 4,9 ppm
$J_{H_1H_2}$ = 5 Hz
pour l'isomère cis: $\underline{H}_1$ δ = 4,7 ppm
$J_{H_1H_2}$ = 7 Hz

*3. Préparation de la [(1H-indényl-2)-3 propyl]-4 pipéridine*

15 g de [(triphénylméthyl-1 pipéridinyl-4)-3 propyl]-2 dihydro-2,3 1H-indénol-1 sont dissous dans un mélange de 75 ml d'une solution aqueuse 5N d'acide chlorhydrique et de 150 ml d'éthanol. La solution est maintenue 16 h à température ambiante, puis chauffée 15 min à 45°C. Le mélange réactionnel est dilué par 150 ml d'eau et l'éthanol est évaporé sous pression réduite. La suspension obtenue est amenée à pH 3 par addition d'hydroxyde de sodium et extraite par 3 fois 100 ml d'éther, puis amenée à pH 10 par addition d'hydroxyde de sodium. L'huile qui relargue est extraite par 3 fois 100 ml d'acétate d'éthyle. Les extraits à l'acétate d'éthyle sont lavés à l'eau, séchés sur sulfate de magnésium et évaporés. Le résidu obtenu (6 g) est fixé sur une colonne contenant 120 g de silice et on élue par un mélange chloroforme/diéthylamine 90/10. On obtient ainsi 5,1 g du produit recherché pur, qu'on transforme en méthanesulfonate par addition d'une solution d'acide méthanesulfonique dans l'acétone. On obtient alors 6 g de méthanesulfonate de [(1H-indényl-2)-3 propyl]-4 pipéridine, qui fond à 152°C.

*Exemple VI: [(Dihydro-2,3 1H-indényl-1)-3 propyl]-4 pipéridine*

3,6 g de chlorhydrate de [(1H-indényl-3)-3 propyl]-4 pipéridine en solution dans 40 ml de méthanol sont hydrogénés à température ambiante, sous une pression d'hydrogène de 1 bar, en présence de 0,36 g de palladium sous forme de charbon palladié à 10% de palladium. Après 1 h, l'hydrogénation est terminée. Le catalyseur est éliminé par filtration et le filtrat est évaporé à sec. Le résidu cristallisé est mis en suspension dans 70 ml d'acétone. Après essorage des cristaux et séchage, on obtient 3,05 g de chlorhydrate de [(dihydro-2,3 1H-indényl-1)-3 propyl]-4 pipéridine, qui fond à 145°C.

*Exemple VII: [(Dihydro-2,3 1H-indényl-2) méthyl]-4 pipéridine*

On opère comme à l'exemple VI, en partant de 4 g de [(1H-indényl-2) méthyl]-4 pipéridine. On obtient 2,9 g de chlorhydrate de [(dihydro-2,3 1H-indényl-2) méthyl]-4 pipéridine, qui fond à 250°C.

*Exemple VIII: [(Dihydro-2,3 1H-indényl-2)-3 propyl]-4 pipéridine*

On opère comme à l'exemple VI, en partant de 2,5 g de [(1H-indényl-2)-3 propyl]-4 pipéridine. On obtient 2,2 g de [(dihydro-2,3 1H-indényl-2)-3 propyl]-4 pipéridine, dont le méthanesulfonate fond à 134°C.

*Exemple IX: [(Dihydro-2,3 1H-indényl-1) méthyl]-4 pipéridine*

*1. Préparation de la [(1H-indényl-3) méthyl]-4 pyridine*

On fait réagir, à 0°C, une solution de 39,6 g de dihydro-2,3 indénone-1 dans 80 ml de tétrahydrofuranne anhydre avec une solution de méthyl-4 pyridine lithiée, obtenue par action de 182 ml d'une solution 2N de butyllithium dans l'hexane sur 31 g de méthyl-4 pyridine dissous dans 300 ml de tétrahydrofuranne anhydre. Une fois la réaction terminée, on ajoute au mélange réactionnel 30 ml d'eau, puis élimine le tétrahydrofuranne et l'hexane par évaporation sous pression réduite. La suspension aqueuse résiduelle est amenée à pH 1 par addition d'une solution aqueuse concentrée d'acide chlorhydrique. Le précipité obtenu est filtré, lavé à l'eau, puis à l'éther et à l'acétone, et séché. On obtient ainsi 25 g de chlorhydrate de [(1H-indényl-3) méthyl]-4 pyridine, qui fond à 210°C.

*2. Préparation de la [(Dihydro-2,3 1H-indényl-1) méthyl]-4 pipéridine*

On hydrogène à la pression atmosphérique et à la température ambiante 35,5 g de chlorhydrate de

[(1H-indényl-3) méthyl]-4 pyridine dans 400 ml d'acide acétique, en présence de 3,5 g d'oxyde de platine Adams comme catalyseur. Au bout de 8 h, on élimine le catalyseur par filtration et le filtrat est évaporé sous pression réduite. On reprend le résidu par 100 ml d'eau et amène la solution aqueuse à pH 11 par addition d'hydroxyde de sodium. On extrait l'huile qui relargue avec du chloroforme. On lave la phase organique à l'eau, la sèche et l'évapore sous pression réduite. On obtient ainsi 31 g du produit recherché sous la forme d'une huile. Ce produit est transformé en méthanesulfonate par addition d'une solution d'acide méthanesulfonique dans l'acétone. Après recristallisation de ce sel dans l'acétonitrile, on obtient 27 g de méthanesulfonate de [(dihydro-2,3 1H-indényl-1) méthyl]-4 pipéridine, qui fond à 134°C.

*Analyse* pour $C_{15}H_{21}N,CH_3SO_3H$:

| | | | |
|---|---|---|---|
| Calculé: | C 61,7 | H 8,04 | N 4,50% |
| Trouvé: | C 61,5 | H 8,10 | N 4,30% |

*Exemple X: [(Méthoxy-5 dihydro-2,3 1H-indényl-1) méthyl]-4 pipéridine*

*1. Préparation de la [(méthoxy-6 1H-indényl-1) méthyl]-4 pyridine*

On opère comme dans la première partie de l'exemple IX, en remplaçant la dihydro-2,3 indénone-1 par la méthoxy-5 dihydro-2,3 indénone-1.

*2. Préparation de la [(méthoxy-5 dihydro-2,3 1H-indényl-1) méthyl]-4 pipéridine*

10,6 g de [(méthoxy-6 1H-indényl-3) méthyl]-4 pyridine dissous dans 100 ml d'acide acétique sont hydrogénés en présence de 1 g d'oxyde de platine Adams, à la pression atmosphérique et à une température de 30°C. Après 5 h, l'hydrogénation est terminée. Le catalyseur est séparé par filtration et le filtrat est évaporé. L'huile résiduelle est dissoute dans 200 ml d'eau. La solution est amenée à pH 5 par addition d'une solution aqueuse de carbonate acide de sodium. Le précipité (traces de produit de départ) est extrait par de l'acétate d'éthyle. La solution aqueuse est amenée à pH 10 par addition d'une solution 10N d'hydroxyde de sodium. L'huile qui relargue est extraite par de l'acétate d'éthyle. La phase organique est lavée à l'eau, séchée sur sulfate de magnésium et évaporée. Le résidu (8,6 g) dissous dans l'acétone est traité par une solution 7N d'acide chlorhydrique dans l'éther (on ajoute la solution d'acide chlorhydrique jusqu'à ce que le pH atteint soit égal à 3). Les cristaux obtenus sont essorés et séchés. On obtient ainsi 9,1 g de chlorhydrate de [(méthoxy-5 dihydro-2,3 1H-indényl-1) méthyl]-4 pipéridine, qui fond à 226°C.

*Exemple XI: [(Chloro-6 dihydro-2,3 1H-indényl-1) méthyl]-4 pipéridine*

*1. Préparation de la [(chloro-5 1H-indényl-3) méthyl]-4 pyridine*

On opère comme dans la première partie de l'exemple IX, en remplaçant la dihydro-2,3 indénone-1 par la chloro-6 dihydro-2,3 indénone-1.

*2. Préparation de la [(chloro-6 dihydro-2,3 1H-indényl-1) méthyl]-4 pipéridine*

On opère comme dans la deuxième partie de l'exemple X, en remplaçant au départ les 10,6 g de [(méthoxy-6 1H-indényl-3) méthyl]-4 pyridine par 10,2 g de [(chloro-5 1H-indényl-3) méthyl]-4 pyridine. On obtient ainsi 8,6 g de chlorhydrate de [(chloro-6 dihydro-2,3 1H-indényl-1) méthyl]-4 pipéridine, qui fond à 222°C.

*Exemple XII: [(Méthyl-6 dihydro-2,3 1H-indényl-1) méthyl]-4 pipéridine*

*1. Préparation de la [(méthyl-5 1H-indényl-3) méthyl]-4 pyridine*

On opère comme dans la première partie de l'exemple IX, en remplaçant la dihydro-2,3 indénone-1 par la méthyl-6 dihydro-2,3 indénone-1.

*2. Préparation de la [(méthyl-6 dihydro-2,3 1H-indényl-1) méthyl]-4 pipéridine*

On opère comme dans la deuxième partie de l'exemple X, en remplaçant au départ les 10,6 g de [(méthoxy-6 1H-indényl-3) méthyl]-4 pyridine par 6,8 g de chlorhydrate de [(méthyl-5 1H-indényl-3) méthyl]-4 pyridine. On obtient ainsi 4,2 g de chlorhydrate de [(méthyl-6 dihydro-2,3 1H-indényl-1) méthyl]-4 pipéridine, qui fond à 262°C.

*Exemple XIII: [(Dihydro-2,3 1H-indényl-1)-2 éthyl]-4 pipéridine*

On hydrogène, à la pression atmosphérique et à la température ambiante, 20 g de [(1H-indényl-3)-2 éthyl]-4 pyridine dans 200 ml d'acide acétique, en présence de 2 g d'oxyde de platine Adams comme catalyseur. Au bout de 3 h, on élimine le catalyseur par filtration et évapore le filtrat sous pression réduite. On redissout le résidu dans 200 ml d'eau, amène la solution aqueuse à pH 10 par addition d'hydroxyde de sodium et extrait l'huile qui relargue au chloroforme. La phase organique est évaporée sous pression réduite. On obtient ainsi 21 g du produit recherché sous la forme d'une huile. Cette huile est transformée en fumarate acide par action de l'acide fumarique au sein de l'éthanol. Après une recristallisation dans l'éthanol, on obtient 13,7 g de fumarate acide de [(dihydro-2,3 1H-indényl-1)-2 éthyl]-4 pipéridine pur, qui fond à 149°C.

*Analyse élémentaire* pour $C_{16}H_{23}N$, $C_4H_4O_4$:

| | | | |
|---|---|---|---|
| Calculé: | C 69,57 | H 7,86 | N 4,06% |
| Trouvé: | C 69,60 | H 7,90 | N 4,10% |

La [(1H-indényl-3)-2 éthyl]-4 pyridine peut être préparée comme indiqué dans le brevet US N° 3300506.

*Exemple XIV: [(Dihydro-2,3 1H-indényl-2)-2 éthyl]-4 pipéridine*

*1. Préparation de la (dihydro-2,3 1H-indényl-2)-1 (pyridyl-4)-2 éthanone-1*

A une solution, refroidie à −72°C et placée sous atmosphère d'azote, de 1,9 g de (dihydro-2,3

1H-indényl-2) carboxylate d'éthyle dans 10 ml de tétrahydrofuranne anhydre on ajoute, goutte à goutte, en 1½ h, une solution de méthyl-4 pyridine lithiée obtenue par action de 10 ml d'une solution 2N de butyllithium dans l'hexane sur 1,95 ml de méthyl-4 pyridine dissous dans 20 ml de tétrahydrofuranne anhydre. L'addition terminée, on ajoute 5 ml d'éthanol et on laisse revenir le mélange réactionnel à température ambiante. On évapore les solvants et ajoute au résidu 50 ml d'une solution aqueuse N d'acide chlorhydrique. L'insoluble est extrait à l'éther et la phase aqueuse résiduelle amenée à pH 7 par addition d'une solution aqueuse de carbonate acide de sodium. L'huile qui relargue est extraite par de l'acétate d'éthyle. L'extrait organique est séché sur sulfate de magnésium et évaporé. On obtient ainsi 2 g de (dihydro-2,3 1H-indényl-2)-1 (pyridyl-4)-2 éthanone-1.

*2. Préparation de la [(dihydro-2,3 1H-indényl-2)-2 éthyl]-4 pyridine*

Les 2 g de (dihydro-2,3 1H-indényl-2)-1 (pyridyl-4)-2 éthanone-1 obtenus dans l'étape précédente sont chauffés à 120°C pendant 10 min avec 1,52 ml d'hydrate d'hydrazine à 85% et 6,5 ml de diéthylèneglycol. On ajoute 1,5 g d'hydroxyde de potassium et porte le mélange réactionnel à 160°C pendant 1 h. On laisse revenir à la température ambiante, ajoute 60 ml d'eau, extrait l'insoluble à l'éther. L'extrait éthéré est séché sur sulfate de magnésium et évaporé. Le résidu huileux (1,6 g) est fixé sur une colonne de silice et on élue avec de l'acétate d'éthyle. On obtient ainsi 1,2 g de [(dihydro-2,3 1H-indényl-2)-2 éthyl]-4 pyridine, qui fond à 59°C.

*3. Préparation de la [(dihydro-2,3 1H-indényl-2)-2 éthyl]-4 pipéridine*

1,2 g de [(dihydro-2,3 1H-indényl-2)-2 éthyl]-4 pyridine dissous dans 12 ml d'acide acétique sont hydrogénés en présence de 0,12 g d'oxyde de platine Adams, à la pression atmosphérique et à température ambiante. Après 6 h, l'hydrogénation est terminée. Le catalyseur est séparé par filtration et le filtrat évaporé. On ajoute à l'huile résiduelle 50 ml d'eau et extrait l'insoluble à l'éther. La solution aqueuse est amenée à pH 9 par addition d'une solution aqueuse 10N d'hydroxyde de sodium. L'huile qui relargue est extraite à l'éther. L'extrait éthéré est lavé à l'eau, séché sur sulfate de magnésium et évaporé. Le résidu (1,1 g) est dissous dans l'éthanol et est traité par une solution 6N d'acide chlorhydrique dans l'éther (on ajoute la solution d'acide chlorhydrique jusqu'à ce que le pH atteint soit égal à 2). Les cristaux obtenus sont essorés et séchés. On obtient ainsi 0,95 g de chlorhydrate de [(dihydro-2,3 1H-indényl-2)-2 éthyl]-4 pipéridine, qui fond à 198°C.

*Propriétés pharmacologiques*

*Activité antidépressive*

L'activité des produits de formule (I) a été démontrée à l'aide du test d'inhibition de la recapture de la sérotonine par des synaptosomes de cerveau de rat (cortex), selon la méthode de Kannengiesser et coll. («Biochem. Pharmacol.», *22*, 73, 1973). Les résultats sont exprimés par une dose inhibitrice 50% ($I_{50}$) qui représente la dose de produit, en micromoles par litre, diminuant de 50% la recapture de la sérotonine.

Les résultats obtenus sont rassemblés dans le tableau:

| Produits | $I_{50}$ (µmol/l) |
|---|---|
| Exemple II | 0,009 |
| Exemple III | 0,05 |
| Exemple VII | 0,4 |
| Exemple IX | 0,4 |
| Exemple X | 0,2 |
| Exemple XI | 0,3 |
| Exemple XII | 0,25 |
| Exemple XIII | 0,004 |
| Exemple XIV | 0,005 |

Les composés de formule (I) sont donc de puissants inhibiteurs de recapture de la sérotonine.

*Propriétés toxicologiques*

Les toxicités aiguës des composés de formule (I) ont été déterminées chez la souris mâle $CD_1$ (Charles River) par voie orale. Les $DL_{50}$ ont été calculées, après 3 d d'observation, par la méthode cumulative de J.J. Reed et H. Münch («Amer. J. Hyg.», *27*, 493, 1938).

Les composés de formule (I) se comportent comme des substances relativement peu toxiques chez la souris, puisque les $DL_{50}$ des composés se situent entre 200 et 1000 mg/kg.

*Utilisation thérapeutique*

Les composés selon l'invention et leurs sels pharmaceutiquement acceptables peuvent être utilisés en thérapeutique humaine, sous forme de comprimés, capsules, gélules, suppositoires, solutions ingérables ou injectables, etc., pour le traitement des états pathologiques engendrés par une perturbation du fonctionnement des systèmes sérotoninergiques, en particulier comme antidépresseurs, comme régulateurs du tonus vasculaire sérotoninodépendant (notamment pour le traitement des migraines) et comme analgésiques.

La posologie dépend des effets recherchés et de la voie d'administration utilisée. Par exemple, par voie orale, elle peut être comprise entre 15 et 250 mg de substance active par jour, avec des doses unitaires allant de 5 à 50 mg.

## Revendications

1. Composés de formule générale:

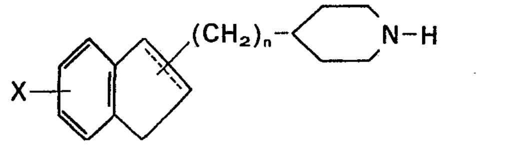

(I)

dans laquelle n est un nombre entier égal à 1, 2 ou 3, X est fixé en position 4, 5, 6 ou 7 sur le cycle

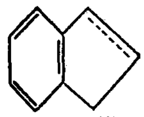

et représente un atome d'hydrogène, un atome d'halogène ou un groupe alkyle, alcoxy ou alkylthio ayant 1 à 4 atomes de carbone, le groupe

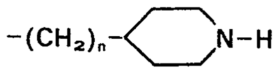

est fixé en position 2 ou 3 sur le cycle

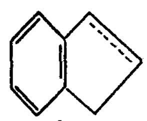

et le trait pointillé représente une deuxième liaison éventuelle, et leurs sels d'addition avec les acides minéraux ou organiques.

2. Composés selon la revendication 1 de formule:

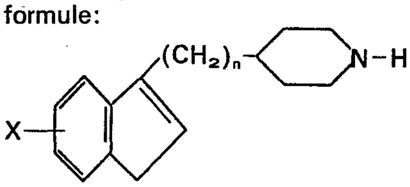

(II)

dans laquelle X et n ont les mêmes significations que dans la revendication 1, et leurs sels d'addition avec les acides minéraux ou organiques.

3. Composés selon la revendication 1 de formule:

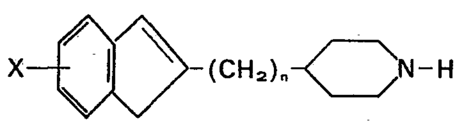

(III)

dans laquelle X et n ont les mêmes significations que dans la revendication 1, et leurs sels d'addition avec les acides minéraux ou organiques.

4. Composés selon la revendication 1 de formule:

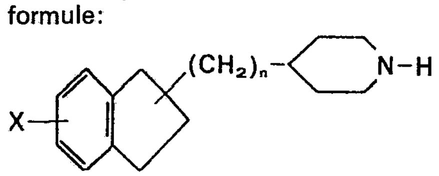

(IV)

dans laquelle X et n ont les mêmes significations que dans la revendication 1, et leurs sels d'addition avec les acides minéraux ou organiques.

5. Composés selon l'une des revendications 1 à 4, caractérisés en ce que X est un atome d'hydrogène.

6. Composé selon la revendication 2 de formule:

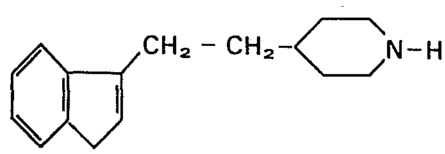

et ses sels d'addition avec les acides minéraux ou organiques.

7. Composé selon la revendication 4 de formule:

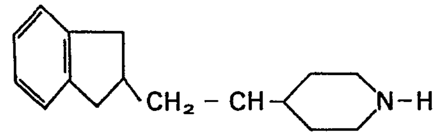

et ses sels d'addition avec les acides minéraux ou organiques.

8. Procédé de préparation des composés selon la revendication 2, caractérisé en ce que l'on fait réagir un composé de formule:

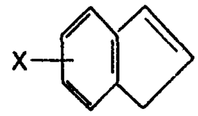

dans laquelle X a la même signification que dans la revendication 2, avec un dérivé métallique de formule RM, dans laquelle M est un métal alcalin et R est un atome d'hydrogène ou un groupe $NH_2$, amino monosubstitué, amino disubstitué, alkyle ou aryle, on fait réagir le composé de formule:

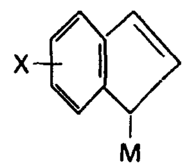

(V)

ainsi obtenu avec un dérivé pipéridinique de formule:

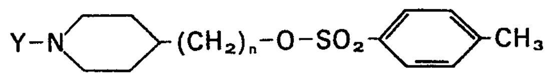

(VI)

dans laquelle n est 1, 2 ou 3 et Y est un groupe protecteur clivable en milieu acide, et on soumet le composé de formule:

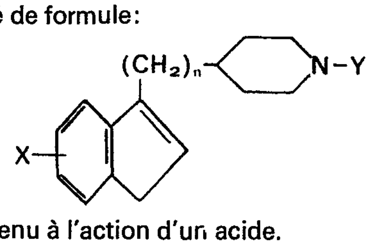

(VII)

ainsi obtenu à l'action d'un acide.

9. Procédé de préparation des composés selon la revendication 3, caractérisé en ce que l'on déshydrate en milieu acide les aminoalcools de formule:

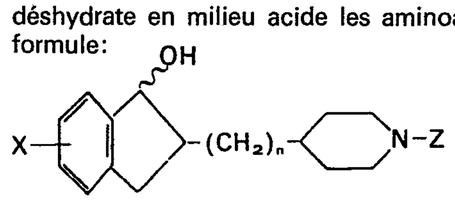

(XI)

dans laquelle X et n ont la même signification que dans la revendication 3 et Z représente un atome d'hydrogène ou un groupe clivable en milieu acide.

10. Procédé de préparation des composés selon la revendication 4, caractérisé en ce que l'on soumet à une hydrogénation catalytique les composés selon les revendications 2 et 3 ou leurs sels.

11. Procédé de préparation des composés selon la revendication 4, caractérisé en ce que l'on soumet à une hydrogénation catalytique les composés de formule:

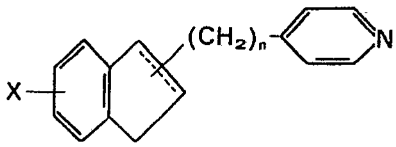

(XII)

dans laquelle X et n ont la même signification que dans la revendication 4, le trait pointillé désigne une deuxième liaison éventuelle et le groupe

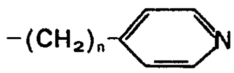

est fixé en position 2 ou 3 sur le cycle

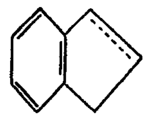

12. Médicament, particulièrement utile comme antidépresseur, antimigraineux et analgésique, caractérisé en ce qu'il contient, en tant que principe actif, un composé répondant à la formule (I) de la revendication 1 ou un sel d'un tel composé avec un acide pharmaceutiquement acceptable.

13. Médicament selon la revendication 12, caractérisé en ce qu'il contient, comme principe actif, le composé de formule:

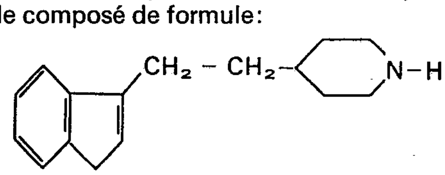

ou un sel de ce composé avec un acide pharmaceutiquement acceptable.

14. Médicament selon la revendication 12, caractérisé en ce qu'il contient, comme principe actif, le composé de formule:

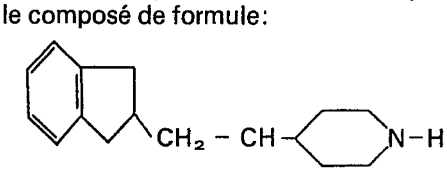

ou un sel de ce composé avec un acide pharmaceutiquement acceptable.

## Patentansprüche

1. Verbindungen der allgemeinen Formel:

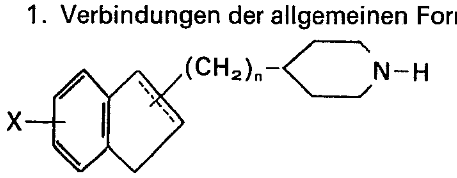

(I)

worin n die ganze Zahl 1, 2 oder 3 bedeutet, X in 4-, 5-, 6- oder 7-Stellung an den Ring

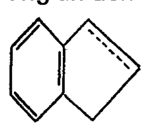

gebunden ist und ein Wasserstoffatom, ein Halogenatom oder eine Alkyl-, Alkoxy- oder Alkylthiogruppe mit 1 bis 4 Kohlenstoffatomen bedeutet, die Gruppe

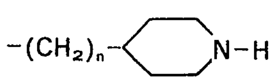

in 2- oder 3-Stellung an den Ring

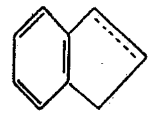

gebunden ist und die gestrichtelte Linie eine gegebenenfalls vorhandene zweite Bindung darstellt, sowie ihre Additionssalze mit mineralischen oder organischen Säuren.

2. Verbindungen nach Anspruch 1 der Formel:

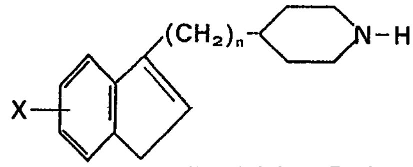

(II)

worin X und n die gleichen Bedeutungen wie im Anspruch 1 haben, sowie ihre Additionssalze mit mineralischen oder organischen Säuren.

3. Verbindungen nach Anspruch 1 der Formel:

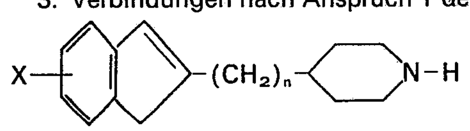

(III)

worin X und n die gleichen Bedeutungen wie im Anspruch 1 haben, sowie ihre Additionssalze mit mineralischen oder organischen Säuren.

4. Verbindungen nach Anspruch 1 der Formel:

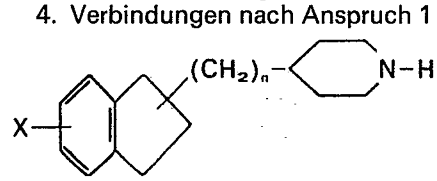

(IV)

worin X und n die gleichen Bedeutungen wie im Anspruch 1 haben, sowie ihre Additionssalze mit mineralischen oder organischen Säuren.

5. Verbindungen nach einem der Ansprüche 1 bis 4, dadurch gekennzeichnet, dass X ein Wasserstoffatom bedeutet.

6. Verbindung nach Anspruch 2 der Formel:

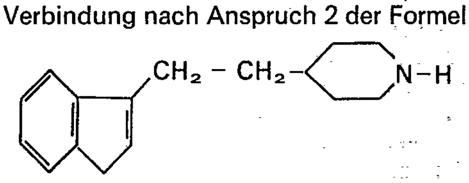

sowie ihre Additionssalze mit mineralischen oder organischen Säuren.

7. Verbindung nach Anspruch 4 der Formel:

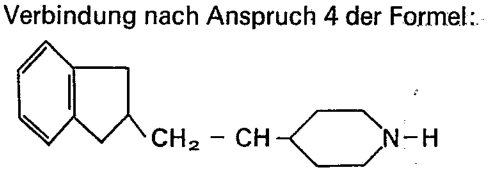

sowie ihre Additionssalze mit mineralischen oder organischen Säuren.

8. Verfahren zur Herstellung der Verbindungen nach Anspruch 2, dadurch gekennzeichnet, dass man eine Verbindung der Formel:

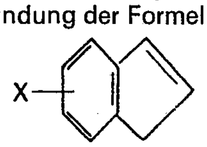

worin X die gleiche Bedeutung wie im Anspruch 2

hat, mit einem Metallderivat der Formel RM, worin M ein Alkalimetall und R ein Wasserstoffatom oder eine $NH_2$-Gruppe, eine monosubstituierte Aminogruppe, eine disubstituierte Aminogruppe, eine Alkylgruppe oder eine Arylgruppe bedeuten, reagieren lässt, die dabei erhaltene Verbindung der Formel:

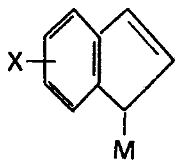

(V)

mit einem Piperidinderivat der Formel:

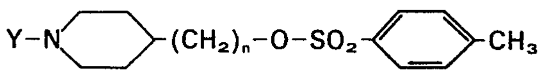

(VI)

worin n die Zahl 1, 2 oder 3 und Y eine in saurem Milieu abspaltbare Schutzgruppe bedeuten, reagieren lässt, und auf die dabei erhaltene Verbindung der Formel:

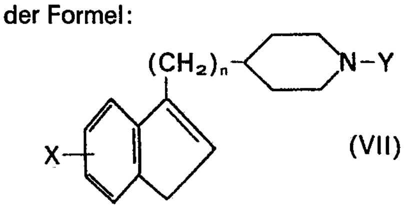

(VII)

eine Säure einwirken lässt.

9. Verfahren zur Herstellung der Verbindungen nach Anspruch 3, dadurch gekennzeichnet, dass man die Aminoalkohole der Formel:

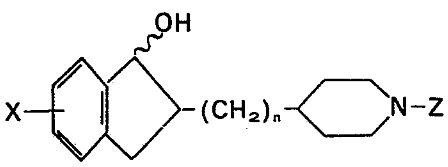

(XI)

worin X und n die gleichen Bedeutungen wie im Anspruch 3 haben und Z ein Wasserstoffatom oder eine in saurem Milieu abspaltbare Gruppe bedeutet, in saurem Milieu dehydratisiert.

10. Verfahren zur Herstellung der Verbindungen nach Anspruch 4, dadurch gekennzeichnet, dass man die Verbindungen der Ansprüche 2 und 3 oder ihre Salze katalytisch hydriert.

11. Verfahren zur Herstellung der Verbindungen nach Anspruch 4, dadurch gekennzeichnet, dass man die Verbindungen der Formel:

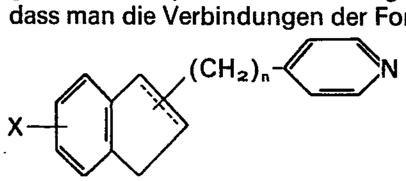

(XII)

worin X und n die gleichen Bedeutungen wie im Anspruch 4 haben, die gestrichelte Linie eine gegebenenfalls vorhandene zweite Bindung bedeutet und die Gruppe

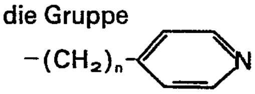

in 2-oder 3-Stellung an den Ring

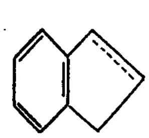

gebunden ist, katalytisch hydriert.

12. Arzneimittel, das insbesondere verwendbar ist als Antidepressivum, Antimigränemittel und Analgetikum, dadurch gekennzeichnet, dass es als Wirkstoff (aktives Prinzip) eine Verbindung der Formel (I) nach Anspruch 1 oder ein Salz einer solchen Verbindung mit einer pharmazeutisch verträglichen Säure enthält.

13. Arzneimittel nach Anspruch 12, dadurch gekennzeichnet, dass es als Wirkstoff (aktives Prinzip) die Verbindung der Formel:

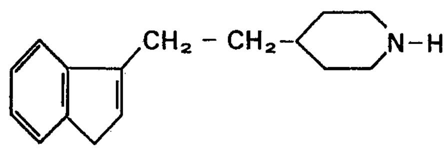

oder ein Salz dieser Verbindung mit einer pharmazeutisch verträglichen Säure enthält.

14. Arzneimittel nach Anspruch 12, dadurch gekennzeichnet, dass es als Wirkstoff (aktives Prinzip) die Verbindung der Formel:

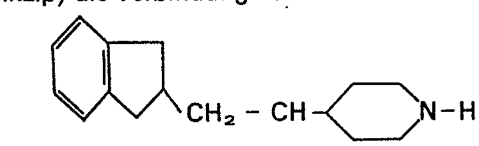

oder ein Salz dieser Verbindung mit einer pharmazeutisch verträglichen Säure enthält.

## Claims

1. Compounds of general formula:

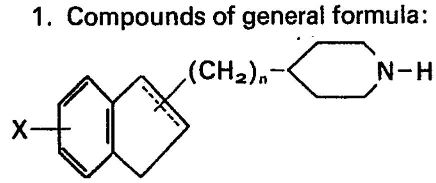

(I)

in which n is a whole number equal to 1, 2 or 3, X is fixed in position 4, 5, 6 or 7 on the ring

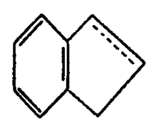

and represents a hydrogen atom, a halogen atom or an alkyl, alkoxy or alkylthio group having 1 to 4 carbon atoms, the group

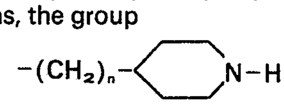

is fixed in position 2 or 3 on the ring

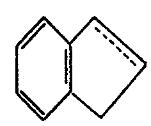

and the dotted line represents a possible second bond, and their addition salts with mineral or organic acids.

2. Compounds according to Claim 1 of formula:

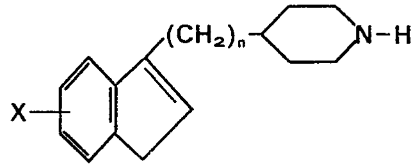

(II)

in which X and n have the same significance as in Claim 1, and their addition salts with mineral or organic acids.

3. Compounds according to Claim 1 of formula:

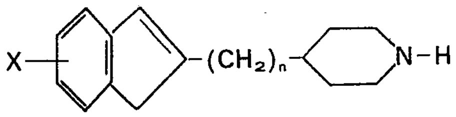

(III)

in which X and n have the same significance as in Claim 1, and their addition salts with mineral or organic acids.

4. Compounds according to Claim 1 of formula:

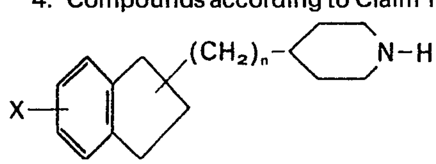

(IV)

in which X and n have the same significance as in Claim 1, and their addition salts with mineral or organic acids.

5. Compounds according to any one of Claims 1 to 4, characterised by the fact that X is a hydrogen atom.

6. Compound according to Claim 2 of formula:

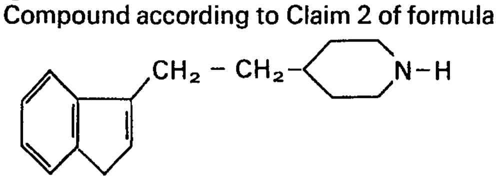

and its addition salts with mineral or organic acids.

7. Compound according to Claim 4 of formula:

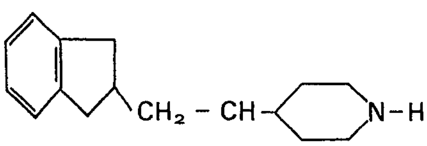

and its addition salts with mineral or organic acids.

8. Process for the preparation of compounds according to Claim 2, characterised in that a compound of formula:

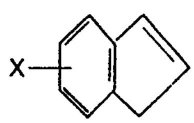

in which X has the same significance as in Claim 2, is reacted with a metal derivative of formula RM, in which M is an alkali metal and R is a hydrogen atom or an $NH_2$, a monosubstituted amino, a disubstituted amino, an alkyl or an aryl group, the compound of formula:

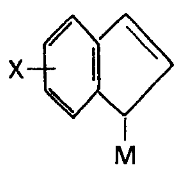

(V)

thus obtained, is reacted with a piperidine derivative of formula:

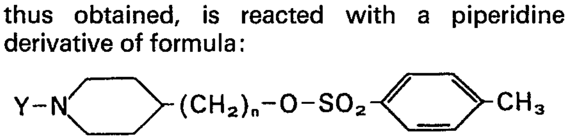

(VI)

in which n is 1, 2 or 3, and Y is a protective group which is cleavable in acid medium, and submitting the compound of formula:

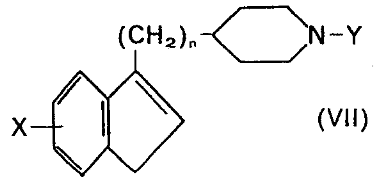

(VII)

thus obtained to the action of an acid.

9. Process for the preparation of compounds according to Claim 3, characterised in that amino alcohols of formula:

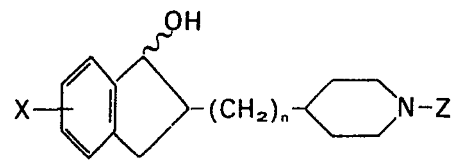

(XI)

in which X and n have the same significance as in Claim 3, and Z represents a hydrogen atom or a group which is cleavable in acid medium, are dehydrated in acid medium.

10. Process for the preparation of compounds according to Claim 4, characterised in that compounds according to Claim 2 and 3 or their salts, are subjected to catalytic hydrogenation.

11. Process for the preparation of compounds according to Claim 4, characterised in that compounds of formula:

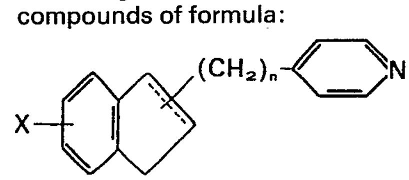

(XII)

in which X and n have the same significance as in Claim 4, the dotted line represents a possible second bond, and the group

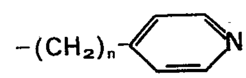

is fixed in position 2 or 3 on the ring 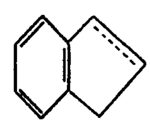

are subjected to catalytic hydrogenation.

12. Medicament, particularly useful as an antidepressant, antimigraine agent and analgesic, characterised in that it contains, as an active principle, a compound corresponding to formula (I) of Claim 1, or a salt of such a compound with a pharmaceutically acceptable acid.

13. Medicament according to Claim 12, characterised in that it contains, as active principle, the compound of formula:

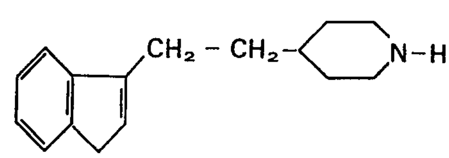

or a salt of this compound with a pharmaceutically acceptable acid.

14. Medicament according to Claim 12, characterised in that it contains, as active principle, the compound of formula:

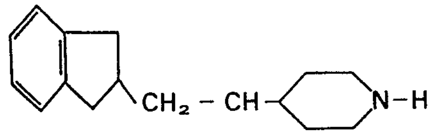

or a salt of this compound with a pharmaceutically acceptable acid.